# EUROPEAN PATENT APPLICATION

(11) **EP 1 719 999 A2**
(43) Date of publication of application: **08.11.2006**
(21) Application number: 06252377.4
(22) Date of filing: 04.05.2006
(51) Int. Cl.: G01N 1/34, A61K 35/28

(54) **Defatted dewatered bone marrow**

(30) Priority: 06.05.2005 US 124501
(71) Applicant: DePuy Spine, Inc., Raynham, MA 02767 (US)
(72) Inventor: Attawia, Mohammed, Canton MA 02021 (US); DiMauro, Thomas, Southboro MA 01722 (US); Holy, Chantal, Raynham MA 02767 (US)
(74) Representative: Belcher, Simon James

(57) **Abstract**

A method of preparing bone marrow aspirate from a patient, comprises obtaining bone marrow aspirate comprising a fat fraction, serum and stem cells from the patient, and removing a portion of the fat fraction of the BMA to obtain a defatted BMA comprising serum and stem cells.

## Description

Bone marrow has been considered as a source of musculoskeletogenic ("MSG") components for producing autologous graft materials useful in the repair/regeneration of musculoskeletal tissues such as bone, cartilage and tendon. Bone marrow aspirate ("BMA") is typically obtained from the patient during surgery by well known techniques and includes the following components set out in Table I below:

**Table I**

| BMA Component | Volume Fraction |
|---|---|
| Plasma | 40-45 vol% |
| Buffy coat fraction (BCF) | 1-10 vol% |
| Red Blood Cells | 45-50 vol% |

The BCF comprises all of the nucleated bone marrow cells ("NBMC"), platelets, proteins and molecules contained within the density band of materials residing between the serum and red blood cell portions of the BMA, as determined by conventional centrifugation of whole BMA. The NBMC component of the BCF typically comprises the following compliment of cell types and approximate concentrations as set out in Table II:

**Table II**

| NBMC type | Relative native concentration (approx) (cells/total NBMC cells) | Absolute concentration (approx) (cells.ml⁻¹) |
|---|---|---|
| Musculoskeletal precursor cells (MSPCs) | <1% | <200,000 |
| Nucleated hematopoitic cells (HCs) | 95-99% | 20 × 10⁶ |
| Reticulocytes (RCs) | <0.1% | <20,000 |
| Endothelial cells (ECs) | <0.1% | <20,000 |

In a first conventional method of using bone marrow for its osteogenic capacity, whole or "fresh" bone marrow is either used directly as a graft material or is combined with a matrix material to produce a bone graft composite. For example, Harada, Bone 9 (1988) 177-183, disclosed a composite comprising whole BMA within a porous matrix of demineralized bone matrix (DBM) contained within a diffusion chamber. However, the diffusion chamber has a semi-permeable membrane that allows the passage of nutrients, and so prevents the influx of cellular components and vasculature critical to osteogenesis. Moreover, as the success of this procedure depends in part upon the native levels of MSPCs in the bone marrow, and such native levels of MSPCs in the patient's bone marrow can sometimes be depleted, the widespread utility of this procedure is limited. Moreover, even at relatively normal native levels of MSPCs, these cells are relatively scarce in fresh bone marrow and so the osteogenic potential of whole bone marrow *per se* is thereby limited.

In a second conventional method, plasma is removed from whole bone marrow, and the remaining mixture comprising the BCF and red blood cells is either used directly as a graft material or combined with a matrix material to produce a bone graft composite. For example, Ohgushi, J.Biomed. Mat.Res. (1990), 24:1563-70 disclosed centrifuging BMA, and using the remaining red cell/BCF fraction as an interstitial fluid within a porous matrix of HA or TCP. As plasma comprises about 45 volume percent ("vol%") of bone marrow aspirate, this method produces only slightly elevated levels of MSPCs (i.e., less than a 2-fold increase) relative to the native level of MSPCs in the fresh bone marrow. In addition, the suspension essentially lacks the soluble or insoluble factors found in plasma such as albumin. Lastly, the presence of red blood cells ("RBCs") in this composition may also cause inhibition of MSPC activity through steric hinderance of surface accessibility and high local iron concentrations following RBC lysis.

In a third conventional method, the buffy coat of the BMA is isolated from the plasma and red blood cell fractions. For example, Connolly et al., JBJS (1989) pp. 684-691, sought to "optimize" the osteogenic potential of BMA, and disclosed isolating fractions of BMA and then using those fractions as graft material in diffusion chambers. Connolly used the following isolation methods:
(a) simple centrifugation followed by removal of the supernatant (i.e., serum) fraction,
(b) isopyknic centrifugation, followed by separate removal of the light cell (buffy coat) and red cell fractions, and
(c) unit gravity centrifugation, followed by separate removal of the light cell (buffy coat) and red cell fractions.

Although Connolly reported that the concentrated light cell (buffy coat) fraction produced by isopyknic centrifugation yielded the greatest level of calcium production within the diffusion chamber, Connolly chose the combined red cell/light cell fraction produced by simple centrifugation (i.e., light cell and red cell fractions) for further study. Moreover, Connolly did not provide a porous substrate carrier material within the diffusion chamber. Lastly, Connolly's examples that utilized the BCF also eliminated the factors present in the plasma fraction of the BMA.

In a fourth conventional method the isolated buffy coat is further fractionated. For example, Budenz et al., Am.J.Anat., 159 (1980), pp. 455-474, discloses isolating fractions of the BCF of bone marrow aspirate in high concentrations, and inserting that concentrated fraction into a diffusion container which is then implanted into rats. The limitations associated with diffusion chambers has been discussed above. Budenz does not disclose using the entire BCF fraction *in toto.* Lastly, Budenz does not disclose a porous substrate carrier material within the diffusion chamber.

In a fifth conventional method, an enriched fraction of MSPCs (relative to all other NMBCs) is combined with a matrix material to produce a bone graft. MSPCs can be enriched by a variety of well-known methods. For example, US Patent No. 6,049,026 ("Muschler '026") discloses passing bone marrow aspirate through a matrix capable of selectively retaining MSPCs. This process produces a composite having enriched amounts of MSPCs (i.e., up to 2.8-fold higher than the native MSPC level found in the same volume of autologous bone marrow). However, this composite is also devoid of the cells, molecules and proteins present in BMA that are not retained by the substrate, and is depleted of other constituents found in BMA, which do not have a high affinity for the substrate. In addition, the process disclosed in Muschler '026 for enriching the MSPCs is inefficient, routinely failing to capture between about 33% and 56% of the MSPCs present in the BMA. Moreover, Muschler discloses optionally washing the MSPC-laden substrate in order to remove any cells which have been only loosely retained, thereby reducing even further the presence of cells which do not have a high affinity for the substrate. Muschler discloses optionally adding to the composite various discrete bioactive constituents such as platelets, cell adhesion molecules (such as collagens), growth factors (such as BMPs), antibodies (such as STRO-1).

Some investigators disclosed *in vitro* culturing of whole or fractionated BMA in an effort to obtain a plentiful and pure population ofMSPCs. For example, Majors. J. Orthop. Res. (1997) 15:546-557, disclosed isolating the BCF of the BMA by centrifugation, culturing the BCF to produce an enriched MSPC population, and staining the MSPCs as a means for assaying the osteoblastic progenitor population within BMA.

WO-A-97/40137 ("Kadiyala") discloses compositions and methods for augmenting bone formation by administering isolated human mesenchymal stem cells with a ceramic material or matrix or by administering human mesenchymal stem cells; fresh, whole marrow or combinations thereof in a resorbable biopolymer that supports their differentiation into their osteogenic lineage. Kadiyala contemplates the delivery of (i) isolated, culture expanded, human mesenchymal stem cells; (ii) freshly aspirated bone marrow; or (iii) their combination in a carrier material or matrix to provide for improved bone fusion area and fusion mass, when compared to the matrix alone. In Example V, discloses a composition comprising a collagen/ceramic composite mixed 50:50 with fresh bone marrow nucleated cells that had been concentrated ten-fold by centrifugation and buffy coat isolation (BMC). The procedure required for producing the culture-expanded, purified MSPCs is a long and arduous one (often requiring about 21 to 56 days), and so can not be performed intra-operatively.

US-5914121 ("Robey") discloses a composition comprising cultured MSPCs and HA/TCP powder, and optionally adding commercially-prepared fibrinogen and thrombin to the composition for the purpose of making fibrin glue.

A few investigators have reported supplementing porous matrices containing concentrated MSG fractions with whole BMA. For example, Walsh, "Autologous Growth Factor Gel (AGF) And Spinal Fusion" 47^{th} Annual Meeting, ORS, February 2001, discloses a graft material comprising a HAP porous matrix, PRP and whole BMA. However, Walsh does not disclose a concentrated, physiologic fraction of fractionated BMA, only whole BMA.

Matsukura, "Concentration of Bone Marrow Derived Osteoprogenitors for Spinal Fusion", Am. Soc. Bone. Min. Res. 22^{nd} Annual Meeting Abstracts, Sept. 2000, discloses a graft material comprising an enriched fraction of MSPCs, whole bone marrow and a porous matrix. Matsukura does not disclose a concentrated, physiologic fraction of fractionated BMA. The enriched fraction of MSPCs taught in Matsukura is not a suspension and so is depleted of the soluble constituents present in the corresponding physiologic fraction of BMA having high levels of MSPCs.

US-A-2002/0161449 ("Muschler II") discloses a composite bone marrow graft material comprising a porous biocompatible implantable matrix, an enriched population of progenitor cells (MSPCs) and a clot material. The clot material can be a blood clot formed from blood, a bone marrow clot, a platelet gel, a platelet concentrate, fibrin clot or a fibrin glue. Since the enriched population of MSPCs is formed by the method taught in Muschler I and so (like Matsukura) is depleted of the soluble constituents present in the corresponding physiologic fraction of BMA having high levels of MSPCs, Muschler II does not disclose a concentrated, physiologic fraction of fractionated bone marrow aspirate BMA.

US-A-2003/0185803 ("Kadiyala") teaches adding specific constituents to buffy coat fractions of BMA.

In sum, the conventional technologies either:
(a) use whole marrow as a source of MSPCs, and so suffer from low MSPC concentrations (such as Walsh),
(b) seek to enrich MSPCs by wholly eliminating other MSG constituents found in the BMA, and so do not have some of the supplemental MSG constituents present in BMA (such as Muschler I),
(c) introduce isolated supplemental MSG constituents into composites having enriched levels of MSPCs, and so have only partially provided the supplemental MSG constituents present in BMA (such as Muschler I), or
(d) add merely whole BMA into composites having enriched levels of MSPCs and so have only unenhanced levels of the supplemental MSG constituents (such as Muschler II and Matsukura)

Moreover there is only a sporadic appreciation in the prior art of the advantages of combining MSG fractions with a porous matrix. For example, there is no disclosure in the prior art of a combination of a physiologic fraction of BMA in combination with a matrix and supplemented with whole BMA.

The present inventors believe that bone marrow has a special role in tissue regeneration and accordingly is comprised of essentially all of the constituents in essentially the appropriate proportions for the regeneration of tissue. Thus, the present inventors believe that composite tissue repair graft materials having improved musculoskeletogenic capabilities should comprise not only enhanced MSPC levels, but also suitable levels of essentially all of the other MSG constituents found in BMA that are thought to play a role in the tissue repair pathway. However, the present inventors have noted that the conventional procedures of concentrating MSPCs deplete or fully eliminate many of the musculoskeletogenic MSG constituents in BMA thought to play a significant role in musculoskeletogenesis. Therefore, the present inventors have concluded that, although the conventional step of concentrating MSPCs from BMA may enhance osteogenesis in one respect (by enhancing MSPC levels), it may also limit tissue repair in a second respect (depleting, and sometimes wholly eliminating, important supporting MSG constituents from BMA). Accordingly, the resulting conventional high-MSPC products possess significant disadvantages.

In light of these considerations, the present inventors sought to identify and eliminate those components of BMA that most likely do not play a major role in the tissue regeneration.

The first component so identified is fat. Fat, which comprises up to about 3-6 volume percent ("v/o") of red marrow and about 71-92 v/o of yellow marrow, is believed to play no major role in tissue regeneration. However, because fat takes up such a large volumetric proportion of BMA, it effectively dilutes the concentration of MSG constituents of BMA that can be placed within a given volume of defect to be regenerated (such as an intervertebral disc space). The present inventors believe that removing the fat portion of the BMA (but without removing the serum or buffy coat constituents) will effectively concentrate the MSG constituents of the BMA and allow the surgeon to place more of the MSG constituents within a given defect space, thereby enhancing tissue regeneration.

Therefore, in accordance with the present invention, there is provided a method of preparing bone marrow aspirate from a patient, comprising:
(a) obtaining bone marrow aspirate comprising a fat fraction, serum and stem cells from the patient,
(b) removing a portion of the fat fraction of the BMA to obtain a defatted BMA comprising serum and stem cells.

The second component so identified is water. Water, which comprises up to about 82-86 v/o of red marrow and about 7-26 v/o of yellow marrow, is believed to be present in amounts far greater than that required for survival of MSC cells. However, because water takes up such a large volumetric proportion of BMA, it effectively dilutes the concentration of MSG constituents of BMA that can be placed within a given volume of defect to be regenerated (such as an intervertebral disc space). The present inventors believe that removing at least some of the water portion of the BMA (but without removing the serum or buffy coat constituents) will effectively concentrate the MSG constituents of the BMA and will allow the surgeon to place more of the MSG constituents within a given defect space, thereby enhancing tissue regeneration.

Therefore, in accordance with the present invention, there is provided a method of preparing bone marrow aspirate from a patient, comprising:
(a) obtaining bone marrow aspirate comprising a water fraction, serum and stem cells from the patient,
(b) removing a portion of the water fraction of the BMA to obtain a dewatered BMA comprising concentrated serum constituents and concentrated stem cells.

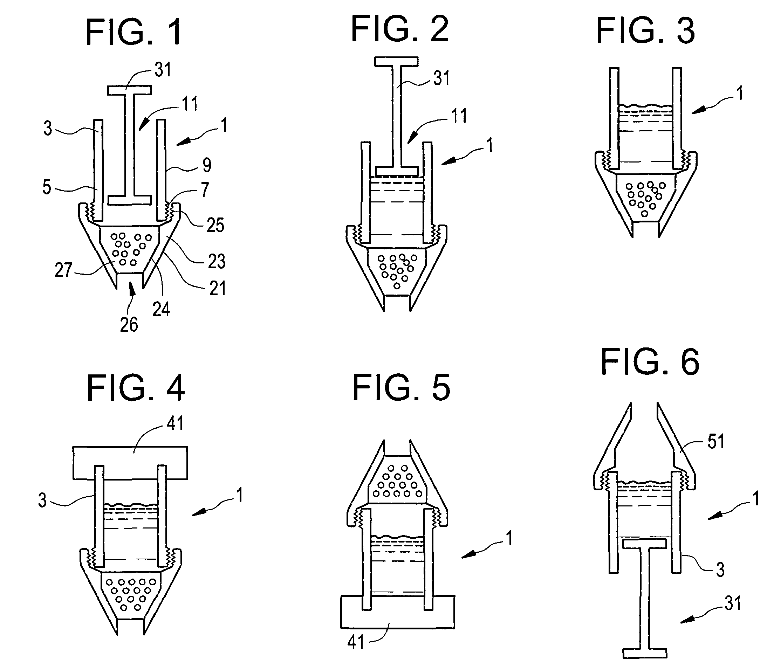

The invention will now be described with reference to the accompanying drawings, in which FIGS. 1-6 disclose use of a preferred syringe of the present invention for obtaining dewatered, defatted BMA.

In some embodiments pertaining to fat removal, the removal of the fat fraction is accomplished by passing the BMA through a filter through which fat does not pass. Preferably, the filter has an average pore size of at least 30 µm, more preferably at least 40 µm. These filter sizes allow the passage of critical BMA cellular components such as stem cells but block the passage of adipocytes (which generally average greater than about 50 µm in an adult).

In some embodiments pertaining to fat removal, the removal of the fat fraction is accomplished by centrifuging the BMA to obtain a fat supernatant and removing the fat supernatant without substantially removing serum. Because fat is clearly the component of BMA having the lowest specific gravity, a low speed centrifugation (such as about 1200 rpm) of the BMA should result in a graduated BMA in which the fat portion resides at the top of the centrifugation column. This fat fraction can then be easily easily by conventional methods without removing substantially any of the serum (that contains constituents important for tissue regeneration).

In some embodiments pertaining to fat removal, the removal of the fat fraction is accomplished by contacting the BMA with a substrate coated with a material that binds adipocytes. When the fat in the BMA contacts the binding material on the substrate surface, it is selectively removed from the BMA without removing any other constituents. In some embodiments thereof, the binding material is adiponectin. Preferably, the BMA is passed through a separation column in which either the walls of the column or beads contained within the column are coated with the binding material.

In some embodiments, at least 25 v/o of the fat is removed from the BMA. In preferred embodiments, at least 50 v/o of the fat is removed from the BMA, more preferably at least 75 v/o.

Since fat often comprises over 50% of BMA, removal of 50% of the fat content of BMA will reduce the effective volume of the BMA by at least about 25%, thereby increasing the concentration of the remaining BMA constituents desirable for tissue regeneration.

In some embodiments, the fat is removed from the bone marrow, the stems cells contained within the fat are substantially isolated from the fat, and those stem cells are then re-added to the remaining bone marrow.

In some embodiments pertaining to water removal, the removal of the water fraction is accomplished by passing water out of the BMA through a filter through which stem cells do not pass. In some embodiments thereof, the BMA is loaded into a separation column having porous sidewalls defined by a porosity that allows water to pass through, but not other BMA constituents. Preferably, the porosity of the side walls has an average pore size of no more than 10 µm, more preferably less than 2 µm.

In some embodiments pertaining to water removal, the removal of the water fraction is accomplished by reverse osmosis.

In some embodiments pertaining to water removal, the removal of the water fraction is accomplished by applying a vacuum to the BMA. In some embodiments thereof, the BMA is placed in a flask having a single opening, tubing is attached to the opening, and a vacuum is drawn through the tubing. The reduced pressure produced within the flask causes the liquid water within the BMA to become a gas which is then removed from the BMA.

In some embodiments pertaining to water removal, the removal of the water fraction is accomplished by applying pressure to the BMA. In embodiments thereof, the BMA is loaded into a simple filter press having a filter size defined by a porosity that allows water to pass through, but not other BMA constituents. Application of pressure to the BMA forces only water through the filter, thereby concentrating the BMA. Preferably, the porosity of the side walls has an average pore size of no more than 10 µm, more preferably less than 2 µm.

In some embodiments of the present invention the removal of the fat and/or water fractions is accomplished without removing red blood cells from the BMA. It is believed that red blood cells provided a somewhat important function of oxygen-binding hemoglobin, which enhances tissue regeneration. However, the present inventors believe that the entire fraction of red blood cells are not needed to accomplish this function. Therefore, in some embodiments, at least a portion of the red blood cells from the BMA. Preferably, when red blood cells are removed, the remaining BMA comprises at least 10 v/o red blood cells, more preferably at least 20 v/o.

In some embodiments, the defatted and/or dewatered BMA is combined with a porous matrix. Preferably, the porous matrix component of the composite of the present invention has a D₅₀ average pore size, as determined by mercury porosimetry, of at least 20 mm. This porosity makes it sufficient to allow nucleated bone marrow cells to flow there through (i.e., it is a scaffold). The ability of these nucleated cells to pass out of the matrix (and for native nucleated cells to pass into the matrix) allows the MSG to take place smoothly and seamlessly both in and around the substrate.

Preferably, the matrix is made from a biocompatible, implantable graft material. Preferably, it is also resorbable. In some embodiments, the material has a charged surface. Preferably, the composite comprises between about 5-50 vol% matrix and between about 50-95 vol% suspension disposed within the pores formed by the matrix. If the volume fraction of the matrix is less than about 5 vol% (excluding its porosity), then the effect of the matrix as a scaffold is not significant.

In some embodiments, the matrix has a sufficient number of pores or passageways so that the total accessible surface area of the substrate is at least five times greater than a solid object having the same external dimensions. Thus, the preferred total surface area can be achieved by using a substrate which comprises a mass of powder, a mass of granules, a mass of fibers, or a highly porous block of substrate material. Preferably, the average pore size in the matrix is greater that 20 mm, more preferably greater than 50 mm, most preferably greater than 100 mm.

Examples of biocompatible, implantable graft materials having a charged surface include ceramics comprising calcium phosphate such as, for example, hydroxyapatite or tri-calcium phosphate; as well as demineralized bone matrix; or mineralized bone matrix. Other suitable graft materials include polylactic acid, polyglycolic acid, polygalactic acid, polycaprolactone, polyethylene oxide, polypropylene oxide, polysulfone, polyethylene, and polypropylene. Other suitable graft materials are hyaluronic acid, which may be purified with or without crosslinking, bioglass, gelatin and collagen. Particularly suitable graft materials include, for example, isolated mineralized cancellous bone sections, powders or granules of mineralized bone, demineralized cancellous bone sections, powders or granules of demineralized bone, guanidine-HCl extracted demineralized bone matrix, sintered cortical or cancellous bone, coralline hydroxyapatite sold by Interpore under the trade marks Interpore 500, or Interpore 200, ProOsteon 500R and granular ceramics such as that incorporated into the bone graft substitute sold by Zimmer under the trade mark Collagraft, filamentous collagen or gelatin sponges such as those sold under the trade marks Gelfoam and Helistat, and deproteinized bone sold by Geistlich Pharma AG-Switzerland under the trade mark BioOss.

The porous matrix can be as disclosed in US-5776193. This porous matrix maintains structural integrity and porosity after implant for a period sufficient to augment the bone replacement process. The matrix comprises mineralized fibrillar insoluble collagen, collagen derivative or modified gelatin, bound with a binder. The minerals comprise particulate calcium phosphate immobilized within the matrix and having a particle size less than about 5 µm. The resulting product is lyophilized, crosslinked, dried and sterilized to form a porous matrix. The matrix may be used as a grafting material and/or a delivery vehicle for osteogenic growth factor. The matrix may be mixed with autogenous bone marrow and implanted for bone regeneration.

The bone grafting matrix is produced using a water-insoluble biodegradable collagen, collagen derivative or modified gelatin. The gelatin will be modified to be insoluble in aqueous environments. The collagen may come from mineralized or unmineralized collagen sources, usually unmineralized collagen sources. Thus, the collagen may come from bone, tendons, skin, or the like, preferably Type I collagen which involves a combination of two strands of a₂ and one al collagen chains. The collagen may be from a young source, e.g., calf, or a mature source, e.g., cow of two or more years. The source of the collagen may be any convenient human or animal source, mammalian or avian, and may include bovine, porcine, equine, chicken, turkey, or other domestic source of collagen. The insoluble collagenous tissue which is employed will normally be dispersed in a medium at an elevated pH, using at least about pH 8, more usually about pH 11-12. Commonly, sodium hydroxide is employed, although other hydroxides may be used, such as other alkali metal hydroxides or ammonium hydroxide.

Native collagen may be utilized in accordance with the present invention. Native collagen contains regions at each end which do not have the triplet glycine sequence. These regions (the telopeptides) are thought to be responsible for the immunogenicity associated with most collagen preparations. The immunogenicity can be mitigated by the removal of these regions to produce atelopeptide-collagen by digestion with proteolytic enzymes, such as trypsin and pepsin.

The concentration of collagen for mineralization will generally be in the range of about 0.1 to 10 weight percent, more usually from about 1 to 5 weight percent. The collagen medium will generally be at a concentrate of the base in the range of about 0.0001 to 0.1N. The pH is generally maintained during the course of the reaction in the range of about 10 to 13, preferably about 12.

Insoluble, fibrillar collagen is preferably used and can be prepared by routine methods. Typically, this can be accomplished with by first mixing with isopropanol (IPA), diethyl ether, hexane, ethyl acetate, or other suitable solvent, and separating the collagen. The pH is typically lowered to about 3, then cooled to about 4°C, and allowed to swell. The resulting slurry may be homogenized until the desired viscosity is attained.

The homogenized slurry is mixed with solvent, agitated, and the pH is raised to about 7. The fibrillar collagen is separated, rinsed with deionized water, and lyophilized. To produce mineralized fibrillar collagen, the purified insoluble collagen fibrils may be homogenized, placed in a reactor where calcium chloride (typically, 0.05M) and tribasic sodium phosphate (typically, 0.03M) are introduced at a controlled rate with stirring. Sodium hydroxide is used to adjust pH at 11.0 +/- 0.5 as needed during this process. After mineralization, the collagen is rinsed with deionized water or phosphate buffer, combined with the binder and the pH is adjusted within a range of 7.5 +/- 1.5. A method of addition of phosphate and calcium ions is disclosed in US-5231169.

The calcium phosphate may contain other ions, such as carbonate, chloride, fluoride, sodium or ammonium. The presence of carbonate results in a product having the properties of dahllite (carbonated hydroxyapatite), while fluoride provides a product having the properties of fluoridated apatite. The weight % of carbonate will usually not exceed 10, while the weight % of fluoride will usually not exceed 2. preferably in the range of 0 to 1. These ions may be present in conjunction with the calcium and/or phosphate source, so long as the ions are compatible and do not result in precipitation in the reagent solutions.

The rate of addition of the calcium and phosphate ions is generally about one hour and no more than about 72 hours in order to achieve the particle size of about 5 µm or less. Generally, the addition period is in the range of about 2 to 18 hours, more usually, in the range of about 4 to 16 hours. Mild temperatures are employed, usually not more than about 40°C, preferably in the range of about 15°C to 30°C. The weight ratio of the collagen to calcium phosphate mineral will generally be in the range of about 8:2 to 1:1, and typically will be about 7:3.

Other non-collagenous proteins or factors, such as BMPs, TGF-β, calcitonin, etc., may be included in the matrix by adding to the collagen slurry, prior or subsequent to calcium and phosphate addition. The amounts of such additives will generally be in the range of about 0.0001 to 2 weight % based on the biopolymer used as the matrix, such as collagen. The added protein may combine with the mineral as it forms on the collagen, binding the added protein to the collagen.

The amount of collagen present in the mineralized product will generally be from about 80% to 30%. Alternatively, the immobilized calcium phosphate particles may be included in the matrix by mixing particles with the binder used to bind the collagen fibrils.

To form a porous, three-dimensional bone grafting matrix, the mineralized collagen fibers are mixed with a binder. Preferably, purified soluble collagen is used as the binder by first mixing soluble collagen with a solvent, such as isopropanol (IPA), and isolating the collagen. The pH is lowered to about 3.0, then, when the collagen is dissolved, the pH is raised to 5.0 washed twice with the solvent, rinsed with deionized water, sieved, and lyophilized.

Other binders which may be used include, but are not limited to, gelatin, polylactic acid, polyglycolic acid, copolymers of lactic and glycolic acid, polycaprolactone, carboxymethylcellulose, cellulose esters (such as the methyl and ethyl esters), cellulose acetate, dextrose, dextran, chitosan, hyaluronic acid, ficol, chondroitin sulfate, polyvinyl alcohol, polyacrylic acid, polypropylene glycol, polyethylene glycol, water soluble methacrylate or acrylate polymers.

In some embodiments, cell adhesion molecules are bound to the surface of the matrix. The term "cell adhesion molecules" refers collectively to laminins, fibronectin, vitronectin, vascular cell adhesion molecules (V-CAM), intercellular adhesion molecules (I-CAM), tenascin, thrombospondin, osteonectin, osteopontin, bone sialoprotein, and collagens.

Optionally, the matrix has growth factors bound to the surface thereof.

Optionally, the matrix has antibodies that have affinity for connective tissue progenitor stem cells bound to the surface thereof. Suitable antibodies, include by way of example, STRO-1, SH-2, SH-3, SH-4, SB-10, SB-20, and antibodies to alkaline phosphatase. Such antibodies are described in Haynesworth et al., Bone 13:69-80,1992a; Bruder, S. et al. Trans Ortho Res Soc 21:574; 1996; Haynesworth, S. E., et al. Bone 13:69-80; 1992; Stewart, K., et al, J Bone Miner Res 11 (Suppl.):S 142;1996; Flemming J E, et al., in "Embryonic Human Skin. Developmental Dynamics" 212:119-132, 1998, and Bruder S P, et al,. Bone 21(3): 225-235, 1997.

In some embodiments, growth factors may be added to the present invention. As used herein, the term "growth factors" encompasses any cellular product that modulates the growth or differentiation of other cells, particularly connective tissue progenitor cells. The growth factors that may be used in accordance with the present invention include, but are not limited to, members of the fibroblast growth factor family, including acidic and basic fibroblast growth factor (FGF-1 and -2) and FGF-4, members of the platelet-derived growth factor (PDGF) family, including PDGF-AB, PDGF-BB and PDGF-AA; EGFs, members of the insulin-like growth factor (IGF) family, including IGF-I and -II; the TGF-β superfamily, including TGF-β1, 2 and 3 (including rhGDF-5), osteoid-inducing factor (OIF), angiogenin(s), endothelins, hepatocyte growth factor and keratinocyte growth factor; members of the bone morphogenetic proteins (BMPs) BMP-1, (BMP-3); BMP-2; OP-1; BMP-2A, -2B, and -7, BMP-14 ; HBGF-1 and -2; growth differentiation factors (GDFs), members of the hedgehog family of proteins, including indian, sonic and desert hedgehog; ADMP-1; members of the interleukin (IL) family, including IL-1 thru -6; rhGDF-5 and members of the colony-stimulating factor (CSF) family, including CSF-1, G-CSF, and GM-CSF; and isoforms thereof.

In some embodiments, the growth factor is selected from the group consisting of TGF-β, bFGF, and IGF-1. These growth factors are believed to promote regeneration of the nucleus pulposus. Preferably, the growth factor is TGF-β. More preferably, TGF-β is administered in an amount of between 10 ng.ml⁻¹ and 5000 ng.ml⁻¹, more preferably between 50 ng.ml⁻¹ and 500 ng.ml⁻¹, more preferably between 100 ng.ml⁻¹ and 300 ng.ml⁻¹.

Any conventional method of obtaining bone marrow aspirate may be used. In one method, percutaneous access to the anterior or posterior iliac crest is obtained through a large bore needle (i.e., Jamshidi) and syringe. Aspiration of marrow contents into a syringe pre-filled with an anticoagulant such as heparin sodium is performed while pulling the needle backward and out from its deepest point of insertion. Multiple punctures into the bone may be performed in order to obtain aspirations with the smallest amount of contamination of peripheral blood. The aspirated BMA is then dewatered and/or defatted in accordance with the methods described above. The dewatered, defatted BMA is then combined with an osteogenic matrix (available from DePuy Spine Inc under the trade mark Healos) and is placed with a fusion cage into an intervertebral disc space.

### EXAMPLE

The following is a prophetic example of preparing a dewatered, defatted bone marrow preparation.

Referring to FIG. 1, a conventional syringe 1 is shown having a proximal end portion 3, a distal end portion 5 having a thread 7 on its outer surface 9, and a throughbore 11 extending from the proximal end portion 3 to the distal end portion 5. A tapered head 21 having an outer annulus 23 adapted to fit to the distal end portion 5 of the syringe (in this case, via threads 25), and defining an inner wall 24 having a distal opening 26. This taper head 21 is attached to the distal end of a conventional syringe. Within the outer annulus 23 of the tapered head 21 resides a 20 mm filter 27. This filter 27 will allow of the passage of red and white blood cells and serum constituents, but not fat cells, into the bore 11 of the syringe 1.

Now referring to FIG. 2, plunger 31 is withdrawn proximally from the bore 11, and BMA is thereby aspirated into the bore 11 of the syringe 1, resulting in fat-free BMA entering the bore 11 of the syringe 1.

Now referring to FIG. 3, the plunger component 31 of the syringe 1 is removed.

Now referring to FIG. 4, a reverse osmosis chamber 41 is added to the proximal end 3 of the syringe 1.

Now referring to FIG. 5, the syringe 1 is inverted for about 5-10 minutes. This allows water from the BMA to irreversibly enter the reverse osmosis chamber 41.

Now referring to FIG. 6, the tapered head 21 containing the fat fraction is removed from the distal portion of the syringe 1 and the plunger 31 is re-inserted into the proximal end of the syringe bore. A filterless tapered head 51 is then attached to the syringe in the place of the filter tapered head. The syringe is now in condition to deliver the dewatered, defatted BMA to the patient, preferably for the fusion of an intervertebral disc space.

Therefore, in accordance with the present invention, there is provided an apparatus for dewatering and defatting bone marrow aspirate, comprising:
(a) a syringe having a proximal end portion, a distal end portion, and a throughbore extending from the proximal end portion to the distal end portion;
(b) a tapered head having (i) an outer annulus adapted to fit to the distal end portion of the syringe and defining an inner wall , and (ii) a filter adapted to trap fat cells, the filter being attached to the inner wall of the outer annulus of the tapered head.

## Claims

1. A method of preparing bone marrow aspirate from a patient, comprising:
(a) obtaining bone marrow aspirate comprising a fat fraction, serum and stem cells from the patient,
(b) removing a portion of the fat fraction of the BMA to obtain a defatted BMA comprising serum and stem cells.

2. The method of claim 1 wherein the removal of the fat fraction is accomplished by passing the BMA through a filter through which fat does not pass.

3. The method of claim 2 wherein the filter has an average pore size of at least 30 µm.

4. The method of claim 1 wherein the removal of the fat fraction is accomplished by centrifuging the BMA to obtain a fat supernatant and removing the fat supernatant without substantially removing serum.

5. The method of claim 1 wherein the removal of the fat fraction is accomplished by contacting the BMA with a substrate coated with a material that binds adipocytes.

6. The method of claim 1 wherein the removal of the fat fraction is accomplished without removing red blood cells from the BMA.

7. The method of claim 1 which includes the step of removing at least a portion of the red blood cells from the BMA.

8. The method of claim 1 which includes the step of combining the defatted BMA with an osteogenic matrix.

9. The method of claim 1 which includes the step of placing the defatted BMA in a defect within the patient.

10. The method of claim 1 which includes the step of dewatering the defatted BMA.

11. A method of preparing bone marrow aspirate from a patient, comprising:
(a) obtaining bone marrow aspirate comprising a water fraction, serum and stem cells from the patient, and
(b) removing a portion of the water fraction of the BMA to obtain a dewatered BMA comprising concentrated serum constituents and concentrated stem cells.

12. The method of claim 11 wherein the removal of the water fraction is accomplished by passing water out of the BMA through a filter through which stem cells do not pass.

13. The method of claim 12 wherein the filter has an average pore size of no more than 10 µm.

14. The method of claim 11 wherein the removal of the water fraction is accomplished by reverse osmosis.

15. The method of claim 11 wherein the removal of the water fraction is accomplished by applying a vacuum to the BMA.

16. The method of claim 11 wherein the removal of the water fraction is accomplished by applying pressure to the BMA.

17. The method of claim 11 wherein the removal of the water fraction is accomplished without removing red blood cells from the BMA.

18. The method of claim 11 which includes the step of removing at least a portion of the red blood cells from the BMA.

19. The method of claim 11 which includes comprising the step of combining the defatted BMA with an osteogenic matrix.

20. The method of claim 11 which includes comprising the step of placing the defatted BMA in a defect within the patient.

21. An apparatus for dewatering and defatting bone marrow aspirate, comprising:
(a) a syringe having a proximal end portion, a distal end portion, and a throughbore extending from the proximal end portion to the distal end portion; and
(b) a tapered head having (i) an outer annulus adapted to fit to the distal end portion of the syringe and defining an inner wall, and (ii) a filter adapted to trap fat cells, the filter being attached to the inner wall of the outer annulus of the tapered head.
